# EUROPEAN PATENT SPECIFICATION

(11) **EP 4 049 690 B1**
(45) Date of publication and mention of the grant of the patent: **14.08.2024**
(21) Application number: 21159240.7
(22) Date of filing: 25.02.2021
(51) Int. Cl.: A61L 31/02, A61L 31/08, A61L 31/14

(54) **METHOD FOR TREATMENT OF MEDICAL METALS AND THEIR ALLOYS**
VERFAHREN ZUR BEHANDLUNG VON MEDIZINISCHEN METALLEN UND DEREN LEGIERUNGEN
PROCÉDÉ DE TRAITEMENT DE MÉTAUX MÉDICAUX ET LEURS ALLIAGES

(43) Date of publication of application: 31.08.2022
(73) Proprietor: Jozef Stefan Institute, 1000 Ljubljana (SI)
(72) Inventor: JUNKAR, Ita, 1000 Ljubljana (SI); BENCINA, Metka, 1000 Ljubljana (SI); ZAPLOTNIK, Rok, 1000 Ljubljana (SI); MOZETIC, Miran, 1000 Ljubljana (SI); SODIN-SEMRL, SNEZNA, 1000 Ljubljana (SI); LAKOTA, Katja, 1000 Ljubljana (SI); IGLIC, Ales, 1000 Ljubljana (SI)
(74) Representative: Marton, Dan-Robert

(56) References cited:
- WO-A1-2014/087414
- WO-A1-2018/029166
- KR-B1- 101 335 723

## Description

### Field of the invention

The present invention relates to the method for formation of oxide layer on the medical metals and their alloys. In particular, the present invention relates to optimisation of surface properties which allows for modification of the surfaces of metals and metal alloys by formation of nano-structured titanium oxide layer additionally treated with highly reactive oxygen species, which significantly improves biological response of biomaterial.

### Background of the invention

Cardiovascular diseases present serious concern, as it presents the number one cause of death in the western world. For example, in patient with coronary artery disease, such as atherosclerosis the arteries/vessels become narrowed and hardened. If the flow of blood to the heart becomes restricted, stents are used to open/restore narrow vessels, help keep the vessels open in order to allow normal blood flow. Stents are usually meshed tubes made from biocompatible, durable and non-corrosive bare metals (bare metal stents -BMS) such as titanium, 316L stainless steel, Nitinol (an alloy of nickel and titanium) and cobalt-chromium (CoCr). The main risk after stent insertion is high platelet adhesion and activation, which play a major role in thrombus formation (thrombosis). Another issue in relation to stent implantation is uncontrolled proliferation of smooth muscle cells that leads to narrowing of a blood vessel and restricted blood flow (restenosis). With the aim to reduce thrombosis and restenosis after stent implantation, the drug-eluting stents (DES) emerged. DESs are bare metal stents, coated with organic (polymers) or inorganic films that have the ability of releasing antiproliferative drugs, such as rapamycin (sirolimus) and paclitaxel. However, significant problems appeared on DES as these stents highly increase late-stent thrombosis, as normal endothelium growth on DES is inhibited. Recently it was observed that the long-term thrombosis rate in DES presents a huge problem and the rate of death of patients treated with DES compared to BMS is higher. Moreover, the degradation products of polymers can be toxic. The development of stent surfaces strives to reach the biocompatibility level that would overcome these serious clinical problems. Stent/implant surfaces of new generations should firstly and foremost inhibit the adhesion and activation of platelets, and furthermore inhibit proliferation and migration of smooth muscle cells (SMC) without the use of anti proliferation agents, while promote proliferation and migration of endothelial cells.

Many different approaches have been addressed and proposed mainly based on various types of coating procedures. One approach is providing polymeric coatings or nanocrystalline ceramic coatings such as titanium dioxide and hydroxyapatite by plasma/thermal sprays or coatings [Fielding et al. Acta Biomaterialia, 2012, 8, 3144.]. Another one is surface functionalisation with biomolecules (e.g.: proteins, heparin, RGD peptides). The next one is nanostructuring of the surface [Kovach et al., Technology A: Vacuum,S.;Films, 2019, 37, 011301, Coen et al., Applied Surface Science, 2003, 207, 276-286]. Nanostructuring of metallic materials significantly influences cellular adhesion, proliferation and differentiation. Surface nanostructuring significantly affects the adhesion and activation of platelets as well as adhesion of other cell types, even bacteria [Kulkarni et al., Nanotechnology, 2015, 26, 062002.]. Surface nanostructuring can be achieved by various techniques such as anodisation, electrospinning, sandblasting or hydrothermal treatment. For example, crystalline titanium dioxide (TiO₂) nano-arrays were synthesised on the Ti substrate with hydrothermal method in tetramethylammonium hydroxide (TMAOH) solution (Dong X, Tao J, Li Y, Zhu H. Appl. Surf. Sci. 2010;256(8):2532-8). Similarly, Ti nanostructures on the Ti foil were prepared by tetrabutylammonium (TBAOH) hydroxide and shape-control agent diethanolamine (DEA) (Roy N, Park Y, Sohn Y, Pradhan D. Semicond. Sci. Technol. 2015;30(4):044005).

US8319002 discloses than appropriately nano-structured surfaces enhance platelet binding and coagulation. The patent discloses haemostatic elements such as polymers, silicon fibres, silicon dioxide nanofibers and/or glass beads into a highly absorbent, gelling scaffold. In another example, nanostructuring of surfaces was obtained by the use of gas-phase plasma. In another invention US7955512 discloses the textured surface accomplished by the use of gas-phase plasma on medical devices with complex geometries, such as stents.

In patent application WO2014087414A1 the preparation of nano-structured surface of Titanium-based stents by a hydrothermal technique in alkaline medium is disclosed. Such a surface finish enables to enhanced endothelialisation and at the same time inhibit proliferation of smooth muscle cells and platelet adhesion. The method involves hydrothermal treatment in alkaline conditions at elevated temperatures (100-300 °C) for different periods of time (1-10 h). Different nanostructures with increased hydrophilicity on the surface of titanium substrate were achieved, for example rods, needles, pores, etc., and/or polygonal shapes such as hexagons, rectangles, triangles, etc.

In another patent application (WO2017158238A1) super-hydrophilic titanium dioxide coating prepared by different techniques such as sol-gel, pulsed laser deposition, ion implantation, electrospraying, chemical vapour deposition process and also hydrothermal method, is disclosed. It is claimed that such coating improves attachment, adhesion and proliferation of bone and other tissue cells. The titanium coating consists of nanostructures of relatively irregular shapes, with the size above 50 nm.

Yet another patent (CN103334105B) describes a method for preparing a vascular stent on magnesium alloy surface with good corrosion resistance and blood compatibility. The method comprises coating of magnesium alloy by hydrothermal coating and formation of titanium oxide layer consisting of nano-sized flakes. In patent US20140277396A1 hydrothermal coating of magnesium alloy used for stents is disclosed. The coating comprises zirconyl nanolaminates. The hydrothermal treatment is carried out at relatively low temperatures (90-100 °C) for 18-96 h.

Further WO 2018/029166 A1 discloses a method of coating a stent, comprising: providing a metal medical device or device with at least a metal surface; immersing said medical device in a bath; depositing ol a titanium oxide layer on the surface of the metal medical device by means of a anodization technique; removal of said medical device from the bath and installing in a plasma reactor; and exposing of said medical device with deposited titanium oxide layer on the surface of said metal medical device to highly reactive oxygen plasma in a second plasma treatment to sterilize and thereby optimize the titanium oxide coating.

Although various surface modifications of metals and alloys were employed, the implantation of metal-based blood-connecting devices still presents a risk of surfaceinduced thrombosis (formation of a blood clot) and in case of vascular stents also restenosis (narrowing of the blood vessel).

### Technical problem to be solved

Thus, the problem of the present invention is ensuring of biocompatibility of metal medical devices, especially vascular stents.

### Summary of the invention

The present invention provides an innovative approach for modification of medical metals and their alloys through a combination of hydrothermal treatment and treatment with highly reactive gaseous plasma according to claim 1, which enables fabrication of surface with superior morphological and chemical properties, especially appropriate for use as vascular stents. Prior to hydrothermal treatment, the surface is exposed to highly reactive plasma in order to improve the adhesion of titanium oxide coating and provide an appropriate morphology of the substrate. The first plasma treatment is followed by the hydrothermal treatment to deposit a thin film of nano-structured titanium oxide. In the next step, the product is further treated with highly reactive oxygen plasma in order to alter surface crystallinity, improve wettability and increase oxygen concentration.

According to the present invention three subsequent treatments are involved: first plasma treatment, then hydrothermal deposition and finally another plasma treatment, namely the second plasma tretment. The plasma parameters of the first plasma treatment may differ from the second plasma treatment. The disclosed technique enables the formation of stable high quality protective nano-structured titanium oxide layer on the surface of said metallic substrate, which significantly influences the biological response.

Medical metals and alloys treated with the innovative method presented herein exhibit improved hemocompatibility/biocompatibility as the adhesion and activation of platelets is highly reduced on these surfaces, which in turn significantly lowers the risk of thrombosis.

In addition, the innovative approach addresses two other important factors, which influence on the long-term success of stent implants: 1 - proper endothelialisation (proliferation/growth of endothelial cells), which allows normal integration of the stent/implant into the human body, and 2 - inhibited adhesion and proliferation of smooth muscle cells, which cause restenosis. Metal substrates treated by the innovative method disclosed herein promote normal adhesion and growth of human coronary artery endothelial cells (HCAEC) and simultaneously inhibit the adhesion and growth of smooth muscle cells (HCASMC). On the contrary, plain Ti substrates enable moderate endothelialisation, but also provide an appropriate environment for the proliferation of smooth muscle cells, which presents a risk of restenosis.

Reduction of platelet adhesion and activation on the surfaces treated with the method of the invention is achieved through a combination of a first plasma treatment, the hydrothermal treatment, and a second plasma treatment. The first plasma treatment enables appropriate surface finish by assuring for contaminant-free surface and nanostructured topography. The hydrothermal treatment enables deposition of a nanostructured titanium dioxide film. The second plasma treatment causes improved wettability and thus minimisation of HCASMC adhesion. In comparison to untreated Ti substrates, which were shown to stimulate platelet adhesion and activation, the surface according to the disclosed invention herein, significantly reduces its adhesion, aggregation and activation.

Method of the invention addresses the need for improved biocompatibility of materials made from metal alloys, in particular vascular stents, with no exclusion to other medical devices, such as orthopaedic implants. The present invention involves:
Step 1: the first plasma treatment is beneficial for the removal of impurities, activation and nanstructuring of the surface of a metallic substrate, wherein the first plasma treatment comprises removal of impurities, activation and nanostructuring of said surface in order to assure a contaminant-free surface with a nano-structured topography.
Step 2: deposition of titanium dioxide film on the surface of said substrate by the hydrothermal method, which enables the formation of titanium oxide nanostructures of size between 10 and 400 nm, with heights from 10 to 150 nm, and the thickness of the oxide layer between 20 to 1.500 nm.
Step 3: The second plasma treatment, which alters physochemical surface properties of the film deposited in step 2, wherein the second plasma treatment of titanium oxide layer is provided with fluencies of OH or O radicals in the range of 10²² to 10²⁶ m⁻², preferably between 10²⁴ and 10²⁵ m⁻². By means of the second plasma treatment an optimization of the deposited film can be achieved, wherein altering of the surface properties is provided.

Against state of the art, the method of the invention enables the formation of a stable film of titanium oxide consisting of nano-sized particles. The first plasma treatment assures for appropriate size and geometry of the nano-sized particles within the stable film of titanium oxide. Also, it assures for optimal adhesion of the nano-sized particles within the stable film of titanium oxide. Such properties are beneficial since they minimise the risk of peeling of the nano-sized particles. The morphology of the surface after performing the three-stage treatment was examined by scanning electron microscopy (SEM). The biocompatibility of the materials made from Ti substrate treated by the methods of the invention is confirmed by the *in vitro* biological studies, where interaction with platelets, human coronary artery endothelial cells (HCAEC) and human coronary artery smooth muscle cells (HCASMC) was studied. The interactions between Ti substrate and cells was monitored by fluorescence microscopy (FM). Biocompatibility of materials treated according to the three-step procedure improved significantly since the platelet adhesion was minimised. Moreover, the methods of the invention provide a healthy environment for the proliferation of human coronary artery endothelial cells, which provides normal endothelialisation after stent implantation. Finally, the adhesion and growth of smooth muscle cells are inhibited, which lowers the risk of stent-restenosis.

Hence a sufficient biocompatibility of metal medical devices, especially blood connecting devices is provided. Thus according to the present invention the surface modification improves the biological response of medical devices in contact with blood.

### Brief description of the figures

The invention is herein described, by way of example, with reference to the accompanying table/drawings, wherein:

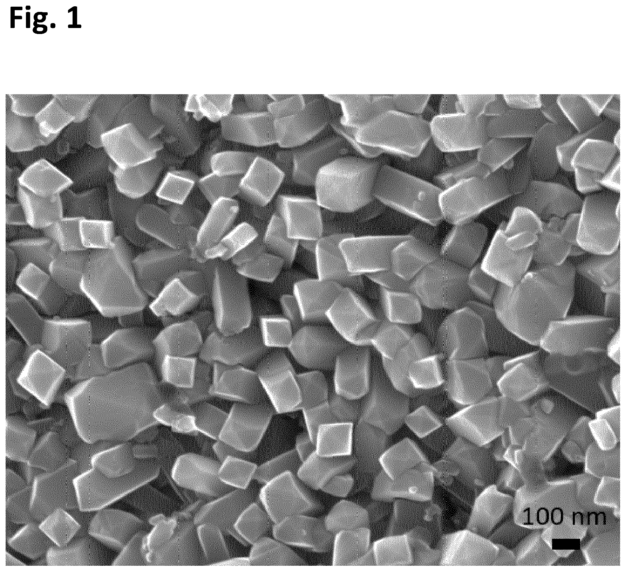

**Fig. 1** presents surface morphology of Ti substrate after the method of invention (treatment step 1, 2 and 3) from SEM image, wherinthe SEM analysis revealed that the morphology of the hydrothermally treated surfaces is nanostructured; composed of nanostructured particles with the size of about 10 to 400 nm.

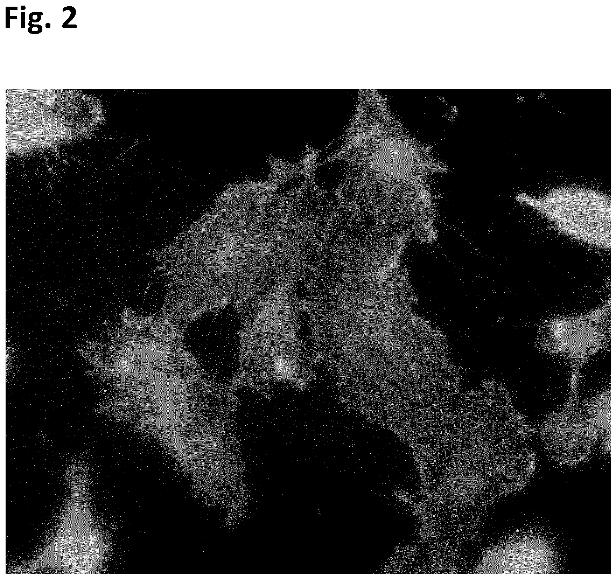

**Fig. 2** shows fluorescence microscopy image of human coronary artery endothelial cells (HCAEC) on Ti substrate after incubation, whereby endothelial cells are well-attached to the surface of Ti substrate, i.e. they are in the elongated form, with non-disrupted cytoplasm.

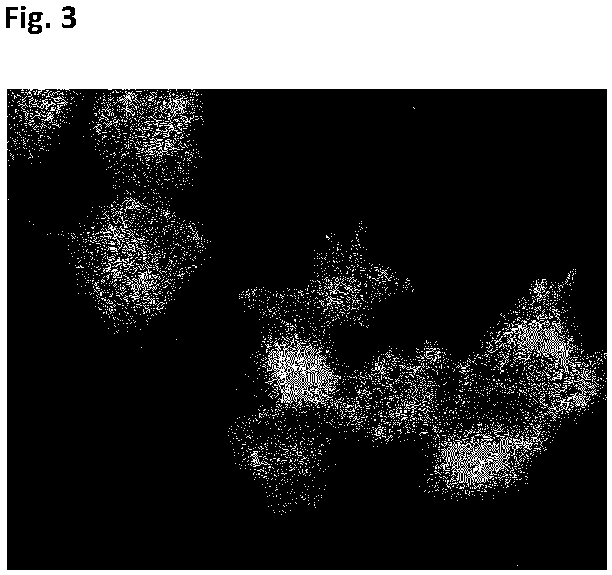

**Fig. 3** shows fluorescence microscopy image of human coronary artery endothelial cells (HCAEC) on hydrothermally treated Ti substrate after incubation, whereby endothelial cells are well-attached to the surface of Ti substrate, with minor destruction of cytoplasm.

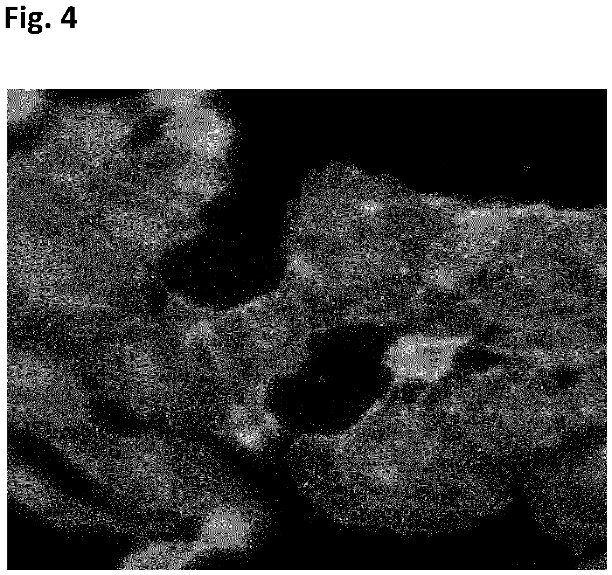

**Fig. 4** shows fluorescence microscopy image of human coronary artery endothelial cells (HCAEC) on Ti substrate treated by the method of invention after incubation, wherein the cells are numerous, well-attached to the surface, in elongated form and "packed" close to each other. This result indicates that surfaces treated with the method of invention promotes superior endothelialisation, which is beneficial for medical devices, especially vascular stents.

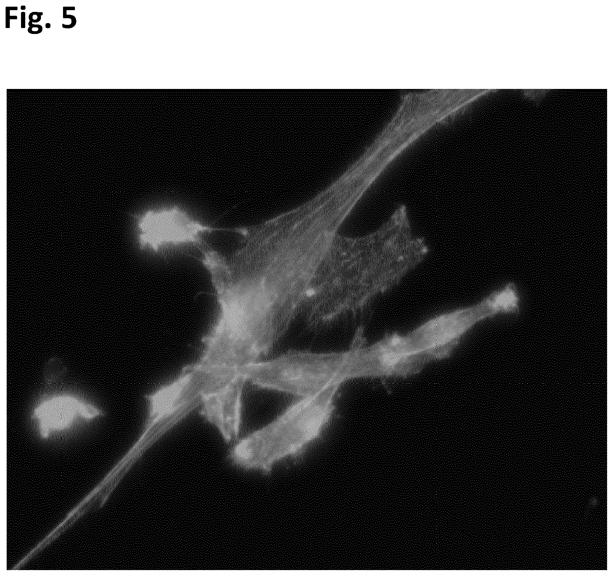

**Fig. 5** shows fluorescence microscopy image of human coronary artery smooth muscle cells (HCASMC) on Ti substrate after incubation. HCASMC are well attached, in elongated form, the proliferation of the cells is high. The results prove that such surfaces provide environment beneficial form HCASMC growth, which indicate higher risk of restenosis in case of vascular stent.

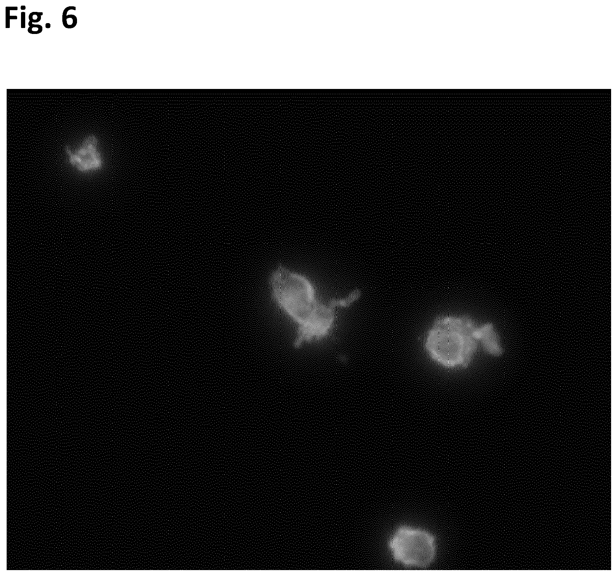

**Fig. 6** shows fluorescence microscopy image of human coronary artery smooth muscle cells (HCASMC) on hydrothermally treated Ti substrate after incubation. HCASMC are rounded, which means the cells are not attached to the surface. High membrane blebbing is also observed. Cells poorly attach and grow on the surface.

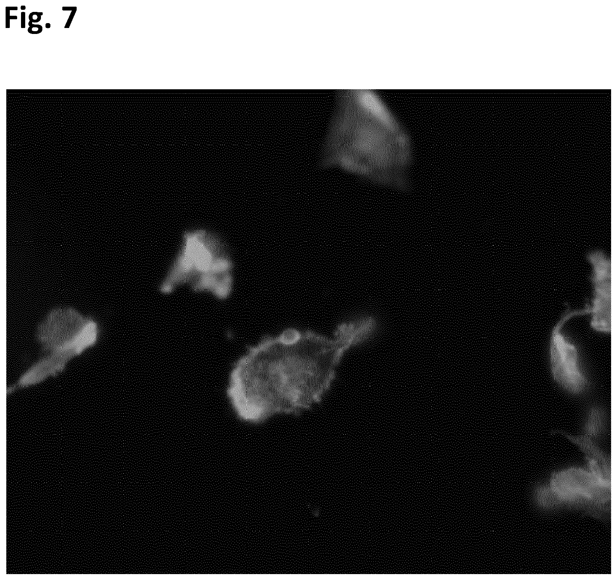

**Fig. 7** shows fluorescence microscopy image of human coronary artery smooth muscle cells (HCASMC) on Ti substrate treated by the method of invention after incubation. The cells are rounded, not-attached to the surface and high membrane blebbing is observed. This result proves that restenosis on surfaces treated with the methods of invention is highly reduced.

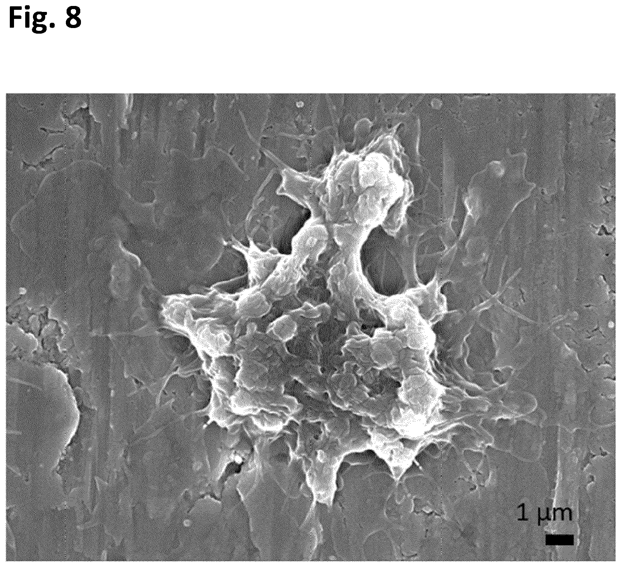

**Fig. 8** shows SEM image of Ti substrate after incubation with whole blood (magnification 5000x). Many platelets observed on the surface in dendritic, spread and fully spread form, which is correlated with high platelet activation on the surface that indicates high risk for thrombosis.

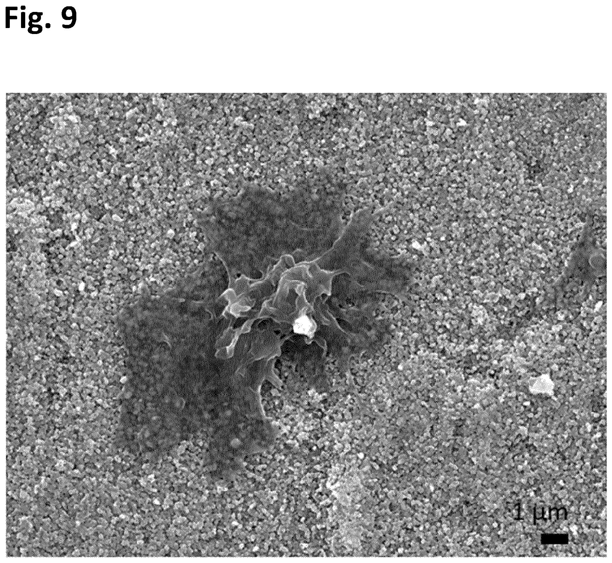

**Fig. 9** shows SEM image of a Ti substrate treated by first plasma and hydrothermal deposition (step 1 and 2), after incubation with whole blood. The surface morphology after hydrothermal treatment is altered and nano-structured morphology which influences on platelate adhesion, which is lower compared to untreated Ti substrated presented in Fig. 8. However platelets detected on these surfaces still adhere and aggregate, which still presents risk of thrombosis.

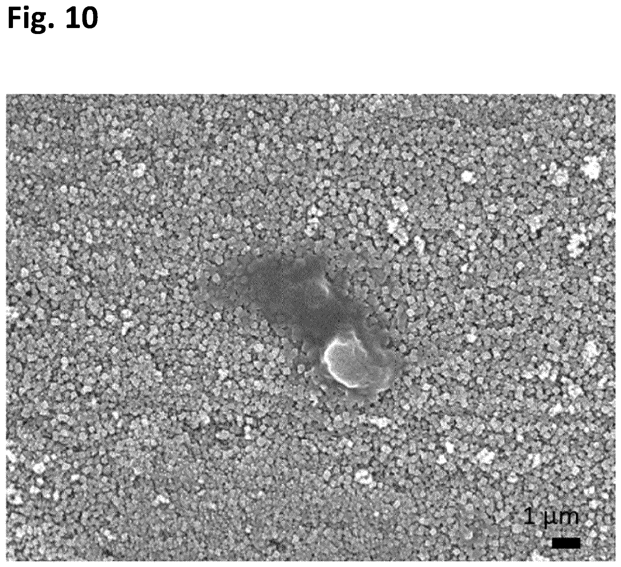

**Fig. 10** shows SEM image of a Ti substrate treated by the innovative three-step method, after incubation with whole blood. There is hardly any platelet detected on the surface and those that are detected are not agregating and are in more round (not activated) form, which lowers the risk of thrombosis.

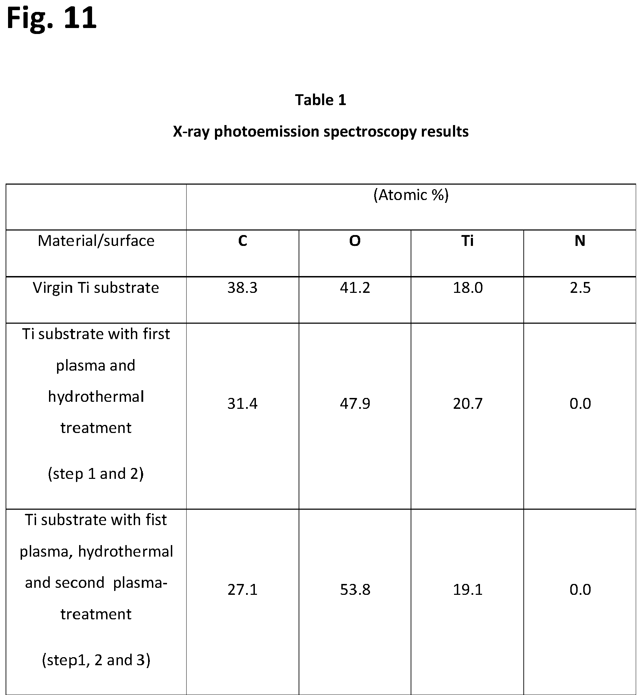

**Fig. 11** presented as **Table 1** chemical composition of the surface of materials examined by X-ray photoelectron spectroscopy. The differences between untreated Ti substrate, hydrothermally treated Ti substrate and Ti substrate treated by the method of invention are presented; the major improvement after treatment with the method of invention is increase in oxygen concentration on the surface of Ti substrate.

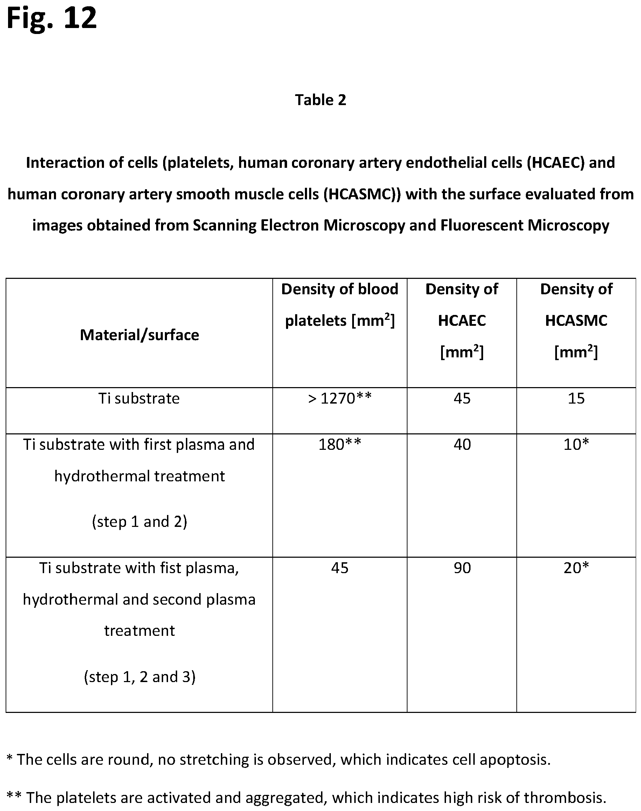

**Fig. 12** presented as **Table 2** interaction of cells (platelets, human coronary artery endothelial cells (HCAEC) and human coronary artery smooth muscle cells (HCASMC)) with the surface evaluated from images obtained from Scanning Electron Microscopy and Fluorescent Microscopy

### Detailed description of the invention

The present invention comprises three steps: 1.) treatment with highly reactive gaseous plasma, 2.) hydrothermal treatment and 3.) surface modification with highly reactive oxygen plasma to ensure surface optimization of the coating. In the first step (step 1) surface is treated by gaseous plasma in order to remove surface impurities, reduce metal oxides and prepare the surface for coating by hydrothermal technique. In the second step (step 2) a thin film of nano-structured titanium oxide is deposited by the hydrothermal method, where nanoparticles with sizes between 30 and 400 nm are formed. In the third step (step 3) surface is exposed to highly reactive oxygen plasma, which causes increase of oxygen concentration on the surface, removes any surface impurities, increases wettability (surface energy) and induces appropriate crystallisation. These surface features significantly influence the biological response, as platelet adhesion and proliferation of smooth muscle cells is reduced, while proliferation of endothelial cells is improved. This is of high importance for medical devices in contact with blood (like vascular stents).

In the first step (step 1) gaseous plasma treatment is used, enabling good adhesion of the thin film of nano-structured titanium oxide. The substrate could be treated by any plasma suitable for removal of surface impurities, an increase of the surface energy and formation of template for the growth of titanium oxide nanostructures. In a preferred embodiment, step 1 is accomplished by treatment with radiofrequency discharge plasma. The plasma treatment time is between 1 and 1000 s, preferably between 10 and 100 s. Plasma can be sustained at different pressures and discharge powers. In the preferred embodiment, plasma is sustained at the pressure of 1- 100 Pa in a mixture of oxygen and water vapour. Such plasma is rich in OH radicals that effectively remove any organic impurities. Furthermore, such plasma is also rich in UV and VUV radiation which causes photoactivation of the thin oxide surface film. According to the present invention the plasma provided in the plasma chamber correspond to a (intrinsic) UV source showing characteristics as mentioned above. However a separate UV source to fulfil the required effects is conceivable according to the present invention.

Surprisingly optimal results could be reached by a UV source with following parameters: wavelength of 365 nm or below with a surface power density between 2,5 to 8 mW cm⁻², preferably 5 mW cm⁻². Thus optimal photoactivation of the oxide film was provided.

In the second step (step 2) the substrate (medical metal material) is submerged in an aqueous Ti isopropoxide solution with the addition of KOH or NaOH. The synthesis of a thin film of nano-structured titanium oxide on the metallic substrate is accomplished in a dielectric container embodied in a stainless steel vessel. After accomplishing the depostion, the surface is washed with deionised water and dried under the stream of nitrogen. The obtained surface consists of octahedral titanium oxide nanoparticles with a size of 30-400 nm as presented in Fig. 1.

In the final step, the material treated with steps 1 and 2 is further subjected to highly reactive oxygen species according to a second plasma treatment step, where the fluencies of oxygen atoms are in the range of 10²² to 10²⁵ m⁻². The results of XPS spectra presented in Fig. 11 respectively Table 1, which shows that formed titanium oxide layer after all three steps has the lowest degree of carbon and highest degree of oxygen on the surface, which plays an importaint role in biocompatibility/hemocompatibility of the surface. Such a treatment can be performed using a variety of plasma treatments. In a preferred embodiment, the treatment is performed by exposure of the sample to oxygen plasma in the range of pressures between 10 and 1.000 Pa. Such plasma is rich in neutral O-atoms that cause oxidation and crystallization of nanostructured titanium oxide layer without significant heating of the sample or disruption of nanostructured features obtained after hydrothermal treatment. Altered surface layer is beneficial since platelets do not readily attach on such surfaces, which significantly reduces thrombosis. Moreover, such treatment significantly improves the proliferation of endothelial cells and at the same time reduces the proliferation of smooth muscle cells.

The efficacy of the Ti substrates has been established in the present invention through *in vitro* approaches:
a.) evaluation of proliferation of human coronary artery endothelial cells (HUVEC) and human coronary artery smooth muscle cells (HCASMC) on control Ti sample, a sample after performing the steps 1 and 2, and a sample treated by the innovative three-step method.
b.) Evaluation of hemocompatibility by incubation with whole human blood. The control Ti sample, a sample after performing the steps 1 and 2, and a sample treated by the innovative three-step method were probed.

The efficiency of the methods of invention will be demonstrated in the following examples, in which Ti material was used as a substrate. The application of the method of the invention is not limited to Ti-based materials but can also be applied to other medical metals and their alloys, such as nickel (Ni), nickel-titanium/Nitinol (NiTi) cobalt-chromium (CoCr) and stainless steel.

### Example 1: Ti substrate - platelet adhesion and activation, HCAEC/HCASMS adhesion and proliferation

In the example disclosed herein, untreated Ti substrate used for stent was analyzed by XPS in order to obtain information about chemical composition of the surface. The untreated Ti substrate chemical composition is presented in Table 1, Ti substrate consists of about 41.2 at. % of carbon, 38.3 at. % of oxygen and 18 at. % of titanium.

Ti substrate was later analysed by fluorescence microscopy in order to gain information about the interaction with the human coronary artery endothelial cells (HCAEC) and human coronary artery smooth muscle cells (HCASMC). HCAEC were purchased from Lifeline Cell Technology (Frederick, MD, USA) and HCASMC were purchased from ProVitro AG (Berlin, Germany). Cells were plated into 75 cm² flasks (TPP, Trasadigen, Switzerland) at 37 °C in a humidified atmosphere at 5% CO₂ and grown in EGM-2M medium containing 5% fetal bovine serum, following manufacturer's instructions (Lonza, Walkesville, MD, USA). For experiments, sub-confluent cell cultures were used between passages 4 and 6. HCAEC and HCASMC were applied onto untreated Ti substrate, which was cleaned with ethanol and dried under a nitrogen stream. The cells and sample materials were placed into 12-well plates at a density of 15 × 10³ cells per cm² and grown for 3 days. Staining with Fluorescein Phalloidin (Molecular Probes, Thermo Fisher Scientific) was performed following manufacturer's instructions. Cells were washed 2 times for 3 min with PBS, pH 7.4, fixed in 3.7 % formaldehyde solution for 10 min and washed 3x for 3 min with PBS at room temperature. Cells were incubated in detergent 0.1% Triton X-100 for 4 min then washed with PBS 3 times for 3 min. Dye stock was diluted 1:40 in PBS with 1% BSA and applied to HCAEC and SMC for 30 min. Final washing steps were performed 3 times for 3 min with PBS. DAPI (4',6-diamidino-2-phenylindole) staining (Molecular Probes, Thermo Fisher Scientific) was performed following the manufacturer's instructions. Briefly, HCAEC on foil and/or NTs of different diameters were incubated with 300 nM DAPI in PBS for 5 min and washed with PBS, for 3 min at room temperature. SlowFade reagent (Thermo Fisher Scientific, USA) was applied to HCAEC and HCASMC, and a coverslip was fixed on top with clear nail polish. Slides were examined and/or stored in the dark at 4 °C. Images were generated using the fluorescent microscope Nikon eclipse E400 and a digital camera (Nikon Instruments, Dusseldorf, Germany). The analysis was performed with Nikon ACT-1 imaging software.

It can be seen from Fig. 2 that HCAEC cells are readily attached to virgin Ti substrate. Cells are fully spread, in elongated form, without the signs of membrane blebbing. Moreover, HCASMC were readily attached to the surface of the untreated substrate and were in elongated form as seen from Fig. 5. Cells have non-disrupted cytoplasm and are found over whole Ti substrate surface.

The average number of different cells interacting with the surface normalised to 1 mm² is shown in Fig. 12 respectively Table 2, first row (Ti substrate).

The adhesion and activation of platelets on untreated virgin Ti substrate was performed according to the following procedure. Ti substrates were cleaned with ethanol and dried under a nitrogen stream. The blood was taken by vein puncture from a healthy human donor. The blood was drawn into 9 ml tubes with trisodium citrate anticoagulant (Sigma Aldrich). Afterwards, the fresh blood (300 µl) was incubated with Ti substrates in 24 well plates for 30 min at room temperature. After incubation, the blood was removed, and 250 µl of phosphate-buffered saline (PBS) was added to the Ti substrates. Ti substrates were rinsed 3 times with 250 µl PBS in order to remove weakly adherent platelets. Adherent biological material was subsequently fixed with 250 µl of 1 % GA (glutaraldehyde) solution with the addition of 250 µl PBS for 1h at room temperature. Afterwards, the surfaces were rinsed with 250 µl PBS and then dehydrated using a graded ethanol series (50, 70, 80, 90, 100 and again 100 vol. % ethanol) for 5 min and in the last stage in the series (100 vol.% ethanol) for 15 min. Afterwards, the samples were placed in a container with liquid nitrogen and further kept in vacuum overnight. This drying process preserves the natural structure of the sample and avoids surface tension which could be caused by normal drying. The dried samples were subsequently coated with gold/palladium and examined by means of SEM (Carl Zeiss Supra 35 VP). Evaluation of platelet density, adhesion and activation were deduced from SEM images. Results obtained by SEM analysis clearly indicate that platelets readily attach on the surface of Ti substrate with lamellipodia and filopodia and start to aggregate, as shown in Fig. 8. Observed morphology of platelets can be described as fully spread. Such morphology of platelets has a high potential to cause thrombosis.

### Example 2: Plasma pre-treated/ first plasma (step 1) and hydrothermally treated Ti substrate (step 2) : platelet adhesion and activation, HCAEC/HCASMS adhesion and proliferation

Ti substrate used for stent application was first pre-treated by highly reactive plasma. The substrate was put into the reaction chamber that was filled with a mixture of oxygen and water vapour (10:1) at the pressure of 30 Pa. A radiofrequency discharge of power 500 W was used for plasma generation. The substrate was left to react with plasma under discharge conditions for 10 s. After the period of 10 s. After accomplishing the plasma treatment (step 1) the hydrothermal treatment (step 2) took place, where the substrates ware submerged in an aqueous solution of Ti isopropoxide with the addition of KOH till pH reached 10. The synthesis was carried out within the pot made from Teflon, embodied within a stainless steel vessel, in a conventional furnace in an air atmosphere at 200 °C for 24h, whereby the best results according to the present invention were achieved. After sufficient time, the samples were thoroughly washed with deionised water, dried under the stream of nitrogen and additionally in a conventional furnace at 70 °C for 2h. As-prepared samples were subjected to ultrasonication (working frequency was 37 kHz) for 5 min. Surface morphology is presented in Fig. 1., while chemical composition obtained from XPS is presented in Fig. 11 respectively Table 1. The obtained surface consists of octahedral titanium oxide nanoparticles with the size of 10 to 400 nm, height of structures between 10 to 300 nm and the thickness of oxide layer is between 20 to 1.500 nm.

The incubation procedure with HCAEC/HCASMC and whole blood was the same as the one described in Example 1.

Results of interactions between HCAEC cells and plasma pre-treated and hydrothermally treated Ti substrates revealed that the HCAEC show minor membrane disruptions as can be observed in Fig. 3. Cells attached to the surface; however, their proliferation was limited. For the case of HCASMC, significantly lesser amount of cells was found on the surface of Ti substrate treated by plasma followed by hydrothermal deposition (steps 1 and 2), as compared to plain Ti surface. Smooth muscle cells were rounded and not well-attached; the cytoplasm was highly reduced as seen in Fig. 6. Interaction of platelets with surfaces treated after steps 1 and 2 was found not as extensive as for untreated Ti samples. However, aggregation of platelets on the surface can still be observed in Fig. 9. Platelets are in dendritic and spread form. The average number of different cells interacting with the surfacecells normalised to 1 mm² surface is shown in Fig. 12 respectively Table 2, second row (Ti substrate with 1. and 2 treatment step).

### Example 3: Ti substrate treated with the method of invention (steps 1, 2 and 3) - platelet adhesion and activation, HCAEC/HCASMS adhesion and proliferation

The dried hydrothermally treated surfaces used for vascular stents were mounted into a plasma chamber. The chamber was first thoroughly evacuated, then filled with oxygen at the pressure of 100 Pa. Plasma was ignited at the RF power of 200 W for 10 s, so the samples were exposed to a fluence of natural oxygen atoms of 5×10²² m⁻².

Results of chemical composition are presented in Fig. 11 respectively Table 1. The results indicate that after exposure to oxygen reactive species, the morphology of the surfaces treated by the method of the invention is not destructed (surface is identical as hydrothermally treated surface as shown in Fig. 1). Surface is nanostructured, composed of octahedral particles with the size of 10-400 nm.

The incubation procedure with HCAEC/HCASMC and whole blood was the same as the one described in Examples 1 and 2.

HCAEC on the surfaces of Ti substrate treated with the three-step method of invention were well-attached, numerous, elongated and close to each other, which indicates superior proliferation, (Fig. 4), significantly better than only hydrothermally treated surface (steps 1 - 2) as seen in Fig. 3. Results proved that surfaces treated according to the three-step method of the invention are beneficial for medical devices like vascular stents due to rapid endothelialisation and thus improved integration of the implant with the human body. Contrary, highly shrunk smooth muscle cells (HCASMC) with non-typical morphology were found on surfaces treated bythe innovative three-step method (Fig. 7). Results suggest that surfaces treated with the method of invention do not provide sufficient environment for HCASMC adhesion and proliferation. Therefore, such surfaces inhibit the uncontrolled growth of HCASMC after stent implantation, which reduces restenosis.

A SEM image of a sample treated by the three-step method after incubating with blood is presented in Fig. 10. There are almost no platelets detected on the surface, while those that can be found are mainly in round form and not aggregated as shown in Fig. 3. Such surfaces will with much lower probability elicit undesired thrombus reactions in comparison to the surfaces prepared in Example 1 and 2.

The average number of different cells interacting with the surface normalised to 1 mm² is shown in Fig. 12 respectively Table 2, third row (Ti substrate treated according to the innovative procedure, step 1-3).

## Claims

1. A method of coating a metal medical device, especially vascular stent, comprising:
- providing a metal medical device;
- first treatment of said medical device by gaseous plasma (step 1) to enable removal of any surface impurities by oxidising plasma radicals and surface activation of said device by combined effects of oxidising plasma radicals and ultraviolet radiation, wherein said first plasma treatment comprises removal of impurities, activation and nanostructuring of said surface in order to assure a contaminant-free surface with a nano-structured topography;
- immersing said medical device in a hydrothermal bath within 10 to 1.000 s, especially less than six minutes, after the said first treatment by gaseous plasma has been accomplished;
- depositing of a titanium oxide layer (step 2) on the surface of the metal medical device by means of a hydrothermal technique, wherein said hydrothermal technique provides coating of the surface with said nanostructured titanium oxide layer consisting of nanoparticles and/or nanostructures of various morphologies like rods, needles, leaves, pores, pits, octahedral, squares with the dimension between 10 to 400 nm in width, 10 to 300 nm in height and thickness of titanium oxide layer ranging from 20 to 1.500 nm;
- removal of said medical device from the hydrothermal bath and installing in a plasma reactor;
- exposing of said medical device (step 3) with deposited titanium oxide layer on the surface of said metal medical device to highly reactive oxygen plasma in a second plasma treatment to optimise the titanium oxide coating, wherein said second plasma treatment of titanium oxide layer is provided with fluencies of OH or O radicals in the range of 10²² to 10²⁶ m⁻², preferably between 10²⁴ and 10²⁵ m⁻².

2. Method according to claim 1, wherein said first plasma treatment of said medical device surface corresponds to a combination of oxidising radicals and ultraviolet radiation of said device by means of a plasma UV source, said UV source preferably providing a wavelength of 365 nm or below with a surface power density between 2,5 to 8 mW cm⁻², preferably 5 mW cm⁻².

3. Method according to at least one of the preceding claim, wherein said second plasma treatment provides altering of physochemical surface properties of the deposited Titanium layer.

4. Method according to at least one of the preceding claims, said depositing by means of said hydrothermal technique is provided in an alkali environment with a pH value preferably in a range between 9 and 12.5 and a temperature value preferably in a range between 100 and 250°C, preferably between 150 and 200°C.

5. Method according to at least one of the preceding claims, wherein said hydrothermal technique comprises subjecting of said metal medical device to an alkaline environment on the basis of NaOH and/orKOH and/or other similar alkaline agents untill a pH value above 9 is reached.

6. Method according to at least one of the preceding claims, said hydrothermal technique comprising Ti-isoporpoxide or other similar precursor used as a source of Ti ions.

7. Method according to at least one of the preceding claims, wherein said hydrothermal technique is carried out within the temperature range of 150°C to 200 °C, and preferably during 1- 72h, especially between 15-30h, specifically for 24h.

8. Method according to at least one of the preceding claims, said metal medical device comprising titanium, titanium alloys, nickel-titan (Ni-Ti), cobalt-chromium (Co-Cr), copper and its alloys, cobalt-chromium-nickel and their combined alloys, stainless steel, tantalum and its alloys, magnesium and/or aluminium.

9. Medical device, especially vascular stent, treated according to at least one of the preceding claims, said device comprising a titanium oxide layer consisting of nanoparticles and/or nanostructures of various morphologies like rods, needles, leaves, pores, pits, octahedral, squares with the dimension between 10 to 400 nm in width, 10 to 300 nm in height and thickness ranging from 10 to 1.500 nm.

10. Medical device according to claim 9, wherein said device is an implantable medical device, especially a vascular stent.

## Patentansprüche

1. Verfahren zur Beschichtung einer medizinischen Metallvorrichtung, insbesondere eines Gefäßstents, umfassend:
- Bereitstellung eines medizinischen Geräts aus Metall;
- erste Behandlung der medizinischen Vorrichtung durch gasförmiges Plasma (Schritt 1), um die Entfernung jeglicher Oberflächenverunreinigungen durch oxidierende Plasmaradikale und die Oberflächenaktivierung der Vorrichtung durch die kombinierte Wirkung von oxidierenden Plasmaradikalen und ultravioletter Strahlung zu ermöglichen, wobei die erste Plasmabehandlung die Entfernung von Verunreinigungen, die Aktivierung und die Nanostrukturierung der Oberfläche umfasst, um eine kontaminationsfreie Oberfläche mit einer nanostrukturierten Topographie zu gewährleisten;
- Eintauchen des medizinischen Geräts in ein hydrothermisches Bad innerhalb von 10 bis 1.000 Sekunden, insbesondere weniger als sechs Minuten, nachdem die erste Behandlung mit Gasplasma durchgeführt wurde;
- Abscheidung einer Titanoxidschicht (Schritt 2) auf der Oberfläche der metallischen medizinischen Vorrichtung mittels einer hydrothermalen Technik, wobei die hydrothermale Technik eine Beschichtung der Oberfläche mit der nanostrukturierten Titanoxidschicht vorsieht, die aus Nanopartikeln und/oder Nanostrukturen verschiedener Morphologien wie Stäben, Nadeln, Blättern, Poren, Gruben, oktaedrisch, quadratisch mit einer Abmessung zwischen 10 bis 400 nm in der Breite, 10 bis 300 nm in der Höhe und einer Dicke der Titanoxidschicht von 20 bis 1.500 nm besteht;
- Entnahme des Medizinprodukts aus dem Hydrothermalbad und Einsetzen in einen Plasmareaktor;
- Aussetzen der medizinischen Vorrichtung (Schritt 3) mit abgeschiedener Titanoxidschicht auf der Oberfläche der metallischen medizinischen Vorrichtung einem hochreaktiven Sauerstoffplasma in einer zweiten Plasmabehandlung, um die Titanoxidbeschichtung zu optimieren, wobei die zweite Plasmabehandlung der Titanoxidschicht mit Fluenzen von OH- oder O-Radikalen im Bereich von 10²² bis 10²⁶ m⁻² , vorzugsweise zwischen 10²⁴ und 10²⁵ m⁻² , durchgeführt wird.

2. Verfahren nach Anspruch 1, wobei die erste Plasmabehandlung der Oberfläche der medizinischen Vorrichtung einer Kombination aus oxidierenden Radikalen und ultravioletter Strahlung der Vorrichtung mittels einer Plasma-UV-Quelle entspricht, wobei die UV-Quelle vorzugsweise eine Wellenlänge von 365 nm oder darunter mit einer Oberflächenleistungsdichte zwischen 2,5 und 8 mW cm⁻² , vorzugsweise 5 mW cm⁻² bereitstellt.

3. Verfahren nach mindestens einem der vorhergehenden Ansprüche, wobei die zweite Plasmabehandlung eine Veränderung der physikalisch-chemischen Oberflächeneigenschaften der abgeschiedenen Titanschicht bewirkt.

4. Verfahren nach mindestens einem der vorhergehenden Ansprüche, wobei die Abscheidung mittels der hydrothermalen Technik in einer alkalischen Umgebung mit einem pH-Wert vorzugsweise in einem Bereich zwischen 9 und 12,5 und einem Temperaturwert vorzugsweise in einem Bereich zwischen 100 und 250° C, vorzugsweise zwischen 150 und 200° C erfolgt.

5. Verfahren nach mindestens einem der vorhergehenden Ansprüche, wobei die hydrothermale Technik das Aussetzen der metallischen medizinischen Vorrichtung einer alkalischen Umgebung auf der Basis von NaOH und/oder KOH und/oder anderen ähnlichen alkalischen Mitteln umfasst, bis ein pH-Wert über 9 erreicht ist.

6. Verfahren nach mindestens einem der vorangehenden Ansprüche, wobei die hydrothermale Technik Ti-Isopropoxid oder einen anderen ähnlichen Vorläufer umfasst, der als Quelle für Ti-Ionen verwendet wird.

7. Verfahren nach mindestens einem der vorhergehenden Ansprüche, wobei das hydrothermale Verfahren im Temperaturbereich von 150°C bis 200°C und vorzugsweise während 1 - 72h, insbesondere zwischen 15 - 30h, speziell während 24h durchgeführt wird.

8. Verfahren nach mindestens einem der vorhergehenden Ansprüche, wobei die metallische medizinische Vorrichtung Titan, Titanlegierungen, Nickel-Titan (Ni-Ti), Kobalt-Chrom (Co-Cr), Kupfer und seine Legierungen, Kobalt-Chrom-Nickel und deren kombinierte Legierungen, rostfreien Stahl, Tantal und seine Legierungen, Magnesium und/oder Aluminium umfasst.

9. Medizinische Vorrichtung, insbesondere vaskulärer Stent, behandelt nach mindestens einem der vorhergehenden Ansprüche, wobei die Vorrichtung eine Titanoxidschicht umfasst, die aus Nanopartikeln und/oder Nanostrukturen verschiedener Morphologien wie Stäben, Nadeln, Blättern, Poren, Gruben, oktaedrischen, quadratischen Strukturen mit einer Abmessung von 10 bis 400 nm in der Breite, 10 bis 300 nm in der Höhe und einer Dicke von 10 bis 1.500 nm besteht.

10. Medizinische Vorrichtung nach Anspruch 9, wobei die Vorrichtung eine implantierbare medizinische Vorrichtung, insbesondere ein Gefäßstent, ist.

## Revendications

1. Procédé de revêtement d'un dispositif médical métallique, en particulier d'une endoprothèse vasculaire, comprenant:
- la mise à disposition d'un dispositif médical en métal;
- premier traitement dudit dispositif médical par plasma gazeux (étape 1) pour permettre l'élimination de toute impureté de surface par des radicaux plasmatiques oxydants et l'activation de la surface dudit dispositif par les effets combinés des radicaux plasmatiques oxydants et du rayonnement ultraviolet, ledit premier traitement au plasma comprenant l'élimination des impuretés, l'activation et la nanostructuration de ladite surface afin de garantir une surface exempte de contaminants avec une topographie nano-structurée;
- immerger ledit dispositif médical dans un bain hydrothermique dans un délai de 10 à 1.000 s, notamment moins de six minutes, après que ledit premier traitement par plasma gazeux a été effectué;
- dépôt d'une couche d'oxyde de titane (étape 2) sur la surface du dispositif médical métallique au moyen d'une technique hydrothermique, ladite technique hydrothermique permettant de recouvrir la surface avec ladite couche d'oxyde de titane nanostructurée constituée de nanoparticules et/ou de nanostructures de différentes morphologies telles que des tiges, des aiguilles, des feuilles, des pores, des puits, des octaèdres, des carrés d'une dimension comprise entre 10 et 400 nm en largeur, 10 et 300 nm en hauteur et d'une épaisseur de couche d'oxyde de titane comprise entre 20 et 1,500 nm;
- retrait dudit dispositif médical du bain hydrothermal et installation dans un réacteur à plasma ;
- exposition dudit dispositif médical (étape 3) avec la couche d'oxyde de titane déposée sur la surface dudit dispositif médical métallique à un plasma d'oxygène hautement réactif dans un second traitement au plasma pour optimiser le revêtement d'oxyde de titane, dans lequel ledit second traitement au plasma de la couche d'oxyde de titane est fourni avec des courants de radicaux OH ou O dans la plage de 10²² à 10²⁶ m⁻² , de préférence entre 10²⁴ et 10²⁵ m⁻².

2. Procédé selon la revendication 1, dans lequel le premier traitement au plasma de la surface du dispositif médical correspond à une combinaison de radicaux oxydants et de rayonnement ultraviolet dudit dispositif au moyen d'une source UV plasma, ladite source UV fournissant de préférence une longueur d'onde de 365 nm ou moins avec une densité de puissance de surface comprise entre 2,5 et 8 mW cm⁻² , de préférence 5 mW cm⁻² .

3. Procédé selon au moins l'une des revendications précédentes, dans lequel le second traitement au plasma permet de modifier les propriétés physico-chimiques de la surface de la couche de titane déposée.

4. Procédé selon l'une au moins des revendications précédentes, le dépôt au moyen de la technique hydrothermale est effectué dans un environnement alcalin dont le pH est de préférence compris entre 9 et 12,5 et dont la température est de préférence comprise entre 100 et 250° C, de préférence entre 150 et 200° C.

5. Procédé selon au moins l'une des revendications précédentes, dans lequel ladite technique hydrothermique consiste à soumettre ledit dispositif médical métallique à un environnement alcalin à base de NaOH et/ou de KOH et/ou d'autres agents alcalins similaires jusqu'à ce qu'une valeur de pH supérieure à 9 soit atteinte.

6. Procédé selon l'une au moins des revendications précédentes, ladite technique hydrothermale comprenant de l'isopropylate de Ti ou un autre précurseur similaire utilisé comme source d'ions Ti.

7. Procédé selon l'une au moins des revendications précédentes, dans lequel la technique hydrothermale est réalisée à une température comprise entre 150 °C et 200 °C, et de préférence pendant 1 à 72 heures, en particulier entre 15 et 30 heures, et plus spécifiquement pendant 24 heures.

8. Procédé selon l'une au moins des revendications précédentes, ledit dispositif médical métallique comprenant du titane, des alliages de titane, du nickel-titane (Ni-Ti), du cobalt-chrome (Co-Cr), du cuivre et ses alliages, du cobalt-chrome-nickel et leurs alliages combinés, de l'acier inoxydable, du tantale et ses alliages, du magnésium et/ou de l'aluminium.

9. Dispositif médical, notamment endoprothèse vasculaire, traité selon au moins l'une des revendications précédentes, ledit dispositif comprenant une couche d'oxyde de titane constituée de nanoparticules et/ou de nanostructures de morphologies diverses telles que des tiges, des aiguilles, des feuilles, des pores, des puits, des octaèdres, des carrés de dimensions comprises entre 10 et 400 nm en largeur, 10 et 300 nm en hauteur et d'une épaisseur allant de 10 à 1,500 nm.

10. Dispositif médical selon la revendication 9, dans lequel ledit dispositif est un dispositif médical implantable, en particulier une endoprothèse vasculaire.
